# EUROPEAN PATENT SPECIFICATION

(11) **EP 3 113 778 B1**
(45) Date of publication and mention of the grant of the patent: **06.06.2018**
(21) Application number: 15709324.6
(22) Date of filing: 06.03.2015
(51) Int. Cl.: A61K 31/501, A61P 25/00, A61P 25/14

(54) **PYRIDAZINE DERIVATIVES FOR USE IN THE PREVENTION OR TREATMENT OF AN ATAXIC DISORDER**
PYRIDAZINDERIVATE ZUR VORBEUGUNG ODER BEHANDLUNG EINER ATAXISCHEN ERKRANKUNG
DÉRIVÉS DE PYRIDAZINE POUR LEUR UTILISATION DANS LA PRÉVENTION OU LE TRAITEMENT D'UN TROUBLE ATAXIQUE

(30) Priority: 06.03.2014 GB 201403944
(43) Date of publication of application: 11.01.2017
(73) Proprietor: Takeda Pharmaceutical Company Limited, Osaka-shi, Osaka 541-0045 (JP)
(72) Inventor: ALMOND, Sarah, Cambridge CB4 0PZ (GB)
(74) Representative: Jones, Nicholas Andrew
(86) International application number: PCT/GB2015/050654
(87) International publication number: WO 2015/132608

(56) References cited:
- WO-A2-03/047558
- US-A1- 2013 052 281
- ALMOND S L ET AL: "Behavioral and biochemical characterization of a mutant mouse strain lacking d-amino acid oxidase activity and its implications for schizophrenia", MOLECULAR AND CELLULAR NEUROSCIENCES, SAN DIEGO, US, vol. 32, no. 4, 1 August 2006 (2006-08-01) , pages 324-334, XP024908147, ISSN: 1044-7431, DOI: 10.1016/J.MCN.2006.05.003 [retrieved on 2006-08-01]
- HASHIMOTO A ET AL: "Mice lacking d-amino acid oxidase activity display marked attenuation of stereotypy and ataxia induced by MK-801", BRAIN RESEARCH, ELSEVIER, AMSTERDAM, NL, vol. 1033, no. 2, 8 February 2005 (2005-02-08), pages 210-215, XP027735997, ISSN: 0006-8993 [retrieved on 2005-02-08] cited in the application

## Description

The present invention relates to the use of certain inhibitors of the enzyme D-amino acid oxidase in the prevention or treatment of ataxic disorders, particularly Friedreich's ataxia and spinocerebellar ataxias.

Ataxia is a disorder of the central nervous system wherein the patient is unable to coordinate muscles for the execution of voluntary movement. Typical symptoms of ataxia are gait dysfunctions, imbalance, impaired limb co-ordination and altered speech. In most ataxia disorders, the ataxia is due to damage or degeneration of the cerebellar cortex and its afferent or efferent fibre connections; typical affected brain regions are the cerebellum, posterior column, pyramidal tracts and basal ganglia. Damage can occur as a result of injury or illness (as is the case with acquired ataxia) or because the cerebellum or spinal cord degenerates (as is the case with hereditary ataxia).

Acquired ataxia can have a wide range of potential causes, including severe head injury (for example, the type of injury that can occur during a car crash or a fall); bacterial brain infection such as meningitis or encephalitis; viral infection; conditions that disrupt the supply of blood to the brain such as a stroke, haemorrhage or a transient ischaemic attack; cerebral palsy; multiple sclerosis; sustained long-term alcohol misuse; underactive thyroid gland; and cancer.

Hereditary ataxia is caused by genetic abnormalities which may be autosomal recessive, such as the mutations responsible for Friedreich's ataxia and ataxia-telangiectasia, or autosomal dominant such as the mutations responsible for some cases of spinocerebellar ataxia.

Spinocerebellar ataxias (SCAs) are a group of ataxias which result from damage to the cerebellum (Dueñas et al., "Molecular pathogenesis of spinocerebellar ataxias", Brain, 2006, 129, 1357-1370). The cerebellum controls balance and coordination. Therefore individuals affected by SCA often experience a loss of balance and co-ordination and often first present with changes in movement or manner of walking (gait) (H. Y. Zoghbi, "Spinocerebellar ataxias", Neurobiology of Disease, 2000, 7(5), 523-527).

SCAs are genetically inherited. There are currently at least 31 known types of SCA. These are known as SCA 1-8 and SCA 10-32 (there is currently no condition associated with SCA9 but the name has been reserved). SCAs can be categorized into three main groups, according to the type of mutation in the gene. The first of these is the expanded polyglutamine ataxias (SCA 1, 2, 3, 6, 7 and 17). The second is the non-coding repeat ataxias (SCA 8, 10 and 12). The third is the ataxias caused by other gene mutations (SCA 5, 13, 14 and 27) (Soong & Paulson, "Spinocerebellar ataxias: an update", Current Opinion in Neurology, 2007, 20, 438-446).

In the group of expanded polyglutamine spinocerebellar ataxias, the abnormality in the faulty gene relates to CAG sequences coding for the amino acid glutamine. In normal genes, CAG sequences can be repeated from 6-35 times, however in SCA, these repeats are expanded to include 40-100 repeats (Zoghbi, 2000). These repeats are found in the coding regions of the genes resulting in proteins containing long stretches of the amino acid glutamine. These proteins appear to have a 'toxic effect'. The higher the number of CAG repeats, the ealier the onset of SCA and the more severe that the SCA is (Soong & Paulson, 2007).

In the group of non-coding repeat spinocerebellar ataxias, as with the previous group, there are abnormal repeats of nucleotide sequences but the repeats are found in the non-coding regions of the genes and it remains unclear how these abnormalities cause SCA.

Finally, in the third group of spinocerebellar ataxias including SCA 5, 13, 14 and 27, the abnormality is caused by conventional gene mutations such as a deletion or insertion of a nucleotide base or the exchange of one nucleotide base for another which results in the production of the wrong amino acid for a specific protein (Soong & Paulson, 2007). It has been suggested that *N*-methyl-D-aspartate type glutamate (NMDA) receptors and impaired glutamate-mediated signalling are implicated in the pathogenesis and progression of spinocerebellar ataxia in humans and in animal models. D-serine is an endogenous modulator of NMDA receptors and Saigoh et al. ("The stereo-specific effect of D-serine ethylester and the D-cycloserine in ataxic mutant mice", Brain Research, 1998, 808, 42-47) have shown that the D-serine derivatives, D-serine ethylester and D-cycloserine, have an ameliorating effect on ataxia in mice carrying inherited or chemically-induced ataxia mutations and that they elicit an increase in the concentration of endogenous D-serine in the cerebellum.

D-Amino acid oxidase (DAAO) was one of the first enzymes to be described and the second flavoprotein to be discovered in the mid- 1930s. DAAO converts D-amino acids into the corresponding α-keto acids. It does this by catalyzing the dehydrogenation of D-amino acids to their imino counterparts and a reduced flavin-product complex. The reduced flavin is then (re)oxidized by dioxygen to yield FADox and H₂O₂, whereas the imino acid spontaneously hydrolyzes to the keto acid and NH4⁺.

DAAO is present in most organisms and mammalian tissues. One action of DAAO is to catabolise the neurotransmitter D-serine. By inhibiting the actions of this enzyme, it would be expected that the concentration of endogenous D-serine would increase. In this regard, Hashimoto et al. ("Mice lacking D-amino acid oxidase activity displayed marked attenuation of stereotypy and ataxia induced by MK-801", Brain Research, 2005, 1033(2), 210-215) have shown that mutant DAAO -/- mice treated with MK-801 (an NMDA receptor antagonist that chemically induces behaviours including hyperlocomotion, sterotypy and ataxia) display reduced ataxia compared to wild type DAAO +/+ mice similarly treated.

Furthermore, although Published International Application No. WO 03/047558 (Genset S.A.) suggests the use of certain DAAO inhibitors in treating ataxia, the application contains no test data confirming whether or not the inhibitors in question showed any such efficacy.

In one aspect of the present invention, there is provided a compound of formula (I), or a pharmaceutically acceptable salt thereof, 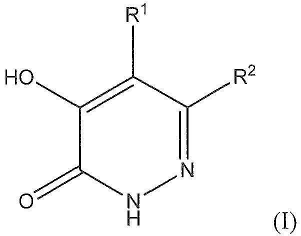 wherein
R¹ represents a hydrogen or fluorine atom or a trifluoromethyl group;
R² represents a group -X-Y-R³;
X and Y each independently represent a bond, an oxygen atom or a group - C(O), -S(O)ₙ, -C(O)NR⁴, -S(O)₂NR⁴, -NR⁴, 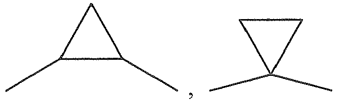 or -CR⁴R⁵-, provided that X and Y cannot both simultaneously represent a bond and provided that if X and Y are both other than a bond, then at least one of X and Y represents -CR⁴R⁵-;
n is 0, 1 or 2;
each R⁴ independently represents a hydrogen atom or a C₁-C₆ alkyl or C₁-C₆ haloalkyl group;
each R⁵ independently represents a hydrogen atom, a C₁-C₆ alkyl or C₁-C₆ haloalkyl group or =CH-;
R³ represents a 3- to 10-membered saturated or unsaturated carbocyclic or heterocyclic ring system, the ring system itself being optionally substituted by at least one substituent selected from halogen, hydroxyl, cyano, oxo, C₁-C₆ alkyl, C₂-C₆ alkenyl, C₁-C₆ haloalkyl, C₁-C₆ hydroxyalkyl, C₁-C₆ alkoxy, C₁-C₆ haloalkoxy, C₁-C₆ alkylthio, C₁-C₆ alkylsulphinyl, C₁-C₆ alkylsulphonyl, C₁-C₆ alkylcarbonyl, C₁-C₆ alkylcarbonyloxy, C₁-C₆ alkoxycarbonyl, amino (-NH₂), -CON(R⁶)₂, C₁-C₆ alkylamino, di-(C₁-C₆ alkyl)amino, C₃-C₆ cycloalkyl, C₃-C₆ cycloalkyloxy, C₃-C₆ cycloalkylmethyl, -[O]ₚ-(CH₂)_{q}-O-R⁷ and a 4- to 6-membered saturated or unsaturated heterocyclic ring (optionally substituted with at least one substituent selected from C₁-C₄ alkyl and C₁-C₄ alkoxy);
each R⁶ independently represents a hydrogen atom or a C₁-C₆ alkyl group;
p is 0 or 1;
q is 1, 2, 3 or 4; and
R⁷ represents a C₁-C₆ alkyl group;
for use in the prevention or treatment of an ataxic disorder, in particular a spinocerebellar ataxic disorder.

In another aspect, the present invention provides a compound of formula (I) or a pharmaceutically acceptable salt thereof as hereinbefore defined for use in reducing the risk of an ataxic disorder.

The compounds of formula (I) and pharmaceutically acceptable salts thereof are known from WO 2013/027000. Compounds of formula (I) have been found from radio-imaging assays to be concentrated in the cerebellar region of the brain and, consequently, to exert their effects primarily in the cerebellum and spinal cord. Therefore, the compounds of formula (I) have the potential to be useful in the treatment of ataxic disorders.

Pharmaceutically acceptable salts of the compounds of formula (I) include acid addition salts which may be formed with inorganic acids and organic acids, e.g., acetate, aspartate, benzoate, besylate, bromide/hydrobromide, bicarbonate/carbonate, bisulfate/sulfate, camphorsulfornate, chloride/hydrochloride, chlortheophyllonate, citrate, ethandisulfonate, fumarate, gluceptate, gluconate, glucuronate, hippurate, hydroiodide/iodide, isethionate, lactate, lactobionate, laurylsulfate, malate, maleate, malonate, mandelate, mesylate, methylsulphate, naphthoate, napsylate, nicotinate, nitrate, octadecanoate, oleate, oxalate, palmitate, pamoate, phosphate/hydrogen phosphate/dihydrogen phosphate, polygalacturonate, propionate, stearate, succinate, sulfosalicylate, tartrate, tosylate and trifluoroacetate salts.

As used herein, the term "treat", "treating" or "treatment" of any disorder refers in one embodiment, to ameliorating the disorder (i.e., slowing or arresting or reducing the development of the disorder or at least one of the clinical symptoms thereof).

In another embodiment "treat", "treating" or "treatment" refers to alleviating or ameliorating at least one physical parameter including those which may not be discernible by the patient.

In yet another embodiment, "treat", "treating" or "treatment" refers to modulating the disorder, either physically, (e.g., stabilisation of a discernible symptom), physiologically, (e.g., stabilisation of a physical parameter), or both.

Thus "treatment" may comprise a reduction in the symptoms associated with an ataxic disorder including, for example, an improvement of gait, balance, limb coordination and/or speech; or an increased period of time between episodes of the ataxic disorder. As used herein, the term "prevention" of any particular disorder refers to the administration of a compound of the present invention to a patient before any symptoms of that disorder are apparent.

As used herein, a patient is "in need of" a treatment if such patient would benefit biologically, medically or in quality of life from such treatment.

The term "a therapeutically effective amount" of a compound of the present invention refers to an amount of the compound of the present invention that will elicit the biological or medical response of a patient, for example, the reduction or inhibition of enzyme activity, or ameliorate symptoms, alleviate conditions, slow or delay progression of a disorder, or prevent a disorder.

In an embodiment of the invention, the compound of formula (I) is selected from
4-Hydroxy-6-(2-phenylethyl)pyridazin-3(2H)-one;
6-[2-(4-Fluorophenyl)ethyl]-4-hydroxypyridazin-3(2H)-one;
4-Hydroxy-6-{2-[5-(trifluoromethyl)pyridin-2-yl]ethyl}pyridazin-3(2H)-one;
6-[(4-Chlorobenzyl)sulfanyl]-4-hydroxypyridazin-3(2H)-one;
4-Hydroxy-6-{2-[6-(trifluoromethyl)pyridin-3-yl]ethyl}pyridazin-3(2H)-one;
6-[2-(3-Fluorophenyl)ethyl]-4-hydroxypyridazin-3(2H)-one;
6-[2-(2-Fluorophenyl)ethyl]-4-hydroxypyridazin-3(2H)-one;
6-[2-(3,5-Difluorophenyl)ethyl]-4-hydroxypyridazin-3(2H)-one;
6-[2-(3,4-Difluorophenyl)ethyl]-4-hydroxypyridazin-3(2H)-one;
4-Hydroxy-6-{2-[3-(trifluoromethoxy)phenyl]ethyl}pyridazin-3(2H)-one;
4-Hydroxy-6-{2-[3-(trifluoromethyl)phenyl]ethyl}pyridazin-3(2H)-one;
4-Hydroxy-6-{2-[5-(trifluoromethyl)pyridin-3-yl]ethyl}pyridazin-3(2H)-one;
6-(2-Cyclohexylethyl)-4-hydroxypyridazin-3(2H)-one;
6-(2-Cyclopropylethyl)-4-hydroxypyridazin-3(2H)-one;
6-(2-Cyclopentylethyl)-4-hydroxypyridazin-3(2H)-one;
4-Hydroxy-6-[2-(4-methoxycyclohexyl)ethyl]pyridazin-3(2H)-one;
6-[2-(2,4-Difluorophenyl)ethyl]-4-hydroxypyridazin-3(2H)-one;
6-{2-[3-(Difluoromethyl)phenyl]ethyl}-4-hydroxypyridazin-3(2H)-one;
6-Benzyl-4-hydroxypyridazin-3 (2H)-one;
6-[2-(3-Chlorophenyl)ethyl]-4-hydroxypyridazin-3(2H)-one;
4-Hydroxy-6-(1-phenylcyclopropyl)pyridazin-3(2H)-one;
4-[2-(5-Hydroxy-6-oxo-1,6-dihydropyridazin-3-yl)ethyl]benzonitrile;
6-[2-(3-Fluoro-4-methylphenyl)ethyl]-4-hydroxypyridazin-3(2H)-one;
6-[2-(4-Fluoro-3-methylphenyl)ethyl]-4-hydroxypyridazin-3(2H)-one;
6-[2-(3,4-Dimethoxyphenyl)ethyl]-4-hydroxypyridazin-3(2H)-one;
6-[2-(4-Chlorophenyl)ethyl]-4-hydroxypyridazin-3(2H)-one;
6-[2-(2-Chlorophenyl)ethyl]-4-hydroxypyridazin-3(2H)-one;
4-Hydroxy-6-{2-[2-(trifluoromethyl)phenyl]ethyl}pyridazin-3(2H)-one;
6-(4-(Difluoromethoxy)phenethyl)-4-hydroxypyridazin-3(2H)-one;
6-(4-(Trifluoromethoxy)phenethyl)-4-hydroxypyridazin-3(2H)-one;
6-(3-(Difluoromethoxy)phenethyl)-4-hydroxypyridazin-3(2H)-one;
6-[1-(4-Fluorophenyl)cyclopropyl]-4-hydroxypyridazin-3(2H)-one;
6-[1-(4-Fluorophenyl)ethyl]-4-hydroxypyridazin-3(2H)-one;
4-Hydroxy-6-{1-[3-(trifluoromethyl)phenyl]ethyl}pyridazin-3(2H)-one;
4-Hydroxy-6-{2-[4-(trifluoromethyl)phenyl]ethyl}pyridazin-3(2H)-one;
6-((Cyclopropylmethyl)(methyl)amino)-4-hydroxypyridazin-3(2H)-one;
6-((Cyclohexylmethyl)(methyl)amino)-4-hydroxypyridazin-3(2H)-one;
6-(3-Chlorobenzyl)-4-hydroxypyridazin-3(2H)-one;
6-(4-Chlorobenzyl)-4-hydroxypyridazin-3 (2H)-one;
6-(Cyclohexylmethyl)-4-hydroxypyridazin-3(2H)-one;
6-(4-Fluorobenzyl)-4-hydroxypyridazin-3(2H)-one;
6-(2-Chloro-6-fluorobenzyl)-4-hydroxypyridazin-3 (2H)-one;
6-(2-Chlorobenzyl)-4-hydroxypyridazin-3(2H)-one;
6-(3-Fluorobenzyl)-4-hydroxypyridazin-3(2H)-one;
6-(2-Fluorobenzyl)-4-hydroxypyridazin-3(2H)-one;
6-(4-Methylbenzyl)-4-hydroxypyridazin-3(2H)-one;
6-(3-Methylbenzyl)-4-hydroxypyridazin-3(2H)-one;
4-Hydroxy-6-(3-(trifluoromethyl)benzyl)pyridazin-3(2H)-one;
4-Hydroxy-6-[2-(oxan-4-yl)ethyl]pyridazin-3(2H)-one;
6-{[(4-Fluorophenyl)methyl](methyl)amino}-4-hydroxy-pyridazin-3(2H)-one;
6-[2-(2,6-Difluorophenyl)ethyl]-4-hydroxy-pyridazin-3(2H)-one;
6-[2-(2-Chloro-6-fluorophenyl)ethyl]-4-hydroxy-pyridazin-3(2H)-one;
6-{[3,5-bis(Trifluoromethyl)phenyl]methyl}-4-hydroxypyridazin-3(2H)-one;
6-(1-Phenylethyl)-4-hydroxypyridazin-3(2H)-one;
6-(Cyclopropylmethyl)-4-hydroxy-2,3-dihydropyridazin-3-one;
4-Hydroxy-6-{1-[4-(trifluoromethyl)phenyl]cyclopropyl}-2,3-dihydropyridazin-3-one;
6-{2-[2-Chloro-4-(trifluoromethyl)phenyl]ethyl}-4-hydroxy-2,3-dihydropyridazin-3-one;
6-{2-[2-Fluoro-4-(trifluoromethyl)phenyl]ethyl}-4-hydroxy-2,3-dihydropyridazin-3-one;
6-{2-[3,5-bis(Trifluoromethyl)phenyl]ethyl}-4-hydroxy-2,3-dihydropyridazin-3-one;
6-{2-[2,4-bis(Trifluoromethyl)phenyl]ethyl}-4-hydroxy-2,3-dihydro-pyridazin-3-one;
6-{2-[3,4-bis(Trifluoromethyl)phenyl]ethyl}-4-hydroxy-2,3-dihydropyridazin-3-one;
4-Hydroxy-6-(3-methyl-4-(trifluoromethyl)phenethyl)pyridazin-3(2H)-one;
4-Hydroxy-6-{2-[2-methyl-4-(trifluoromethyl)phenyl]ethyl}-2,3-dihydropyridazin-3-one;
6-{2-[3,5-Difluoro-4-(trifluoromethyl)phenyl]ethyl}-4-hydroxy-2,3-dihydropyridazin-3-one; and
6-{2-[3-Fluoro-4-(trifluoromethyl)phenyl]ethyl}-4-hydroxy-2,3-dihydropyridazin-3-one.

In another embodiment of the invention, the compound of formula (I) is selected from
6-[2-(4-Fluorophenyl)ethyl]-4-hydroxypyridazin-3(2H)-one;
4-Hydroxy-6-{2-[4-(trifluoromethyl)phenyl]ethyl}pyridazin-3(2H)-one;
6-(4-Chlorobenzyl)-4-hydroxypyridazin-3(2H)-one; and
6-(2-Fluorobenzyl)-4-hydroxypyridazin-3(2H)-one.

The compounds of formula (I) and pharmaceutically acceptable salts thereof may be used on their own but will generally be administered in the form of a pharmaceutical composition in which the formula (I) compound/salt (active ingredient) is in association with a pharmaceutically acceptable adjuvant, diluent or carrier.

Therefore, in a further aspect, the present invention provides a pharmaceutical composition comprising a compound of formula (I) or a pharmaceutically acceptable salt thereof as hereinbefore defined in association with a pharmaceutically acceptable adjuvant, diluent or carrier, for use in the prevention or treatment of an ataxic disorder.

In a still further aspect, the present invention provides a pharmaceutical composition comprising a compound of formula (I) or a pharmaceutically acceptable salt thereof as hereinbefore defined in association with a pharmaceutically acceptable adjuvant, diluent or carrier, for use in reducing the risk of an ataxic disorder.

The compounds of the invention (that is, compounds of formula (I) and pharmaceutically acceptable salts thereof) may also be administered in conjunction with other compounds used for the prevention or treatment of an ataxic disorder.

The present invention therefore further relates to combination therapies wherein a compound of the invention or a pharmaceutical composition comprising a compound of the invention is administered with another therapeutic agent or agents, for the prevention or treatment of an ataxic disorder.

Such therapeutic agents may be selected from D-serine, D-serine ethyl ester, D-cycloserine, amantadine or amantadine hydrochloride ("Symmetrel"), buspirone ("Buspar"), acetazolamide ("Diamox"), topiramate ("Topamax"), divalproex sodium ("Depakote"), L-dopa ("Sinemet"), propranolol ("Inderal"), primidone ("Mysoline"), clonazepam ("Klonopin"), levetiracetam ("Keppra"), carbamazepine ("Tegretol"), gabapentin ("Neurontin"), baclofen ("Lioresal"), ondansetron ("Zofran"), tizanidine ("Zanaflex") and pramipexole ("Mirapex").

The combination therapy may comprise a fixed dose combination of a compound of the invention and one or more other therapeutic agents. Alternatively, the combination therapy may comprise a preparation of a first active ingredient which is a compound of the invention and a preparation of a second active ingredient (for example, a therapeutic agent as previously described) for simultaneous, sequential or separate administration to a patient in need thereof.

The pharmaceutical compositions and combinations according to the invention may be administered systemically, e.g. by oral administration in the form of tablets, capsules, syrups, powders or granules; or by parenteral administration in the form of a sterile solution, suspension or emulsion for injection (including intravenous, subcutaneous, intramuscular, intravascular or infusion); or by rectal administration in the form of suppositories.

Preferably the pharmaceutical compositions and combinations are in unit dosage forms such as tablets, pills and capsules.

For preparing solid compositions such as tablets, a compound of the invention is mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid pre-formulation composition containing a homogeneous mixture of the compound of the invention. When referring to these pre-formulation compositions as homogeneous , it is meant that the compound of the invention (the principal active ingredient) is dispersed evenly throughout the composition so that the composition may be readily sub-divided into equally effective unit dosage forms such as tablets, pills and capsules. This solid pre-formulation composition is then sub-divided into unit dosage forms of the type described above containing from 0.1 to about 500 mg of the compound of the invention. Tablets or pills may be either film-coated or enteric-coated according to methods known in the art.

Pharmaceutical compositions of the invention in liquid form for oral administration include aqueous solutions, suitably flavoured syrups, aqueous or oil suspensions, and flavoured emulsions with edible oils such as cottonseed oil, sesame oil, coconut oil, peanut oil or soybean oil. Suitable dispersing or suspending agents for aqueous suspensions include synthetic and natural gums such as tragacanth, acacia, alginate, dextran, sodium carboxymethylcellulose, methylcellulose, polyvinyl-pyrrolidone or gelatin.

Conventional procedures for the selection and preparation of suitable pharmaceutical formulations are described in, for example, "Pharmaceutics - The Science of Dosage Form Design", M. E. Aulton, Churchill Livingstone, 1988.

It will be appreciated that the amounts of the compound of the invention and, if present, one or more other therapeutic agents required for use in the prevention or treatment of an ataxic disorder will vary not only with the particular compound of the invention or other therapeutic agent(s) selected but also with the route of administration, the nature of the condition being treated, and the age and condition of the patient, and will ultimately be at the discretion of the patient's physician or pharmacist. If administered orally, the daily dosage of the compound of the invention may be in the range from 0.01 micrograms per kilogram body weight (µg/kg) to 100 milligrams per kilogram body weight (mg/kg).

The present invention will now be further explained by reference to the following illustrative example.

### Example 1

### Beam Walking Test

Friedreich ataxia (FRDA) is an autosomal recessive neurodegenerative disorder, caused by a GAA repeat expansion mutation within intron 1 of the FXN gene, resulting in reduced level of frataxin protein. Normal individuals have 5 to 40 GAA repeat sequences, whereas affected individuals have approximately 70 to greater than 1000 GAA repeat sequences. Frataxin is a mitochondrial protein involved in iron-sulphur cluster and heme biosynthesis. The reduction in frataxin expression leads to oxidative stress, mitochondrial iron accumulation and consequential cell death, primarily in the neurons of the dorsal root ganglia and the dentate nucleus of the cerebellum. FRDA, which is the most commonly inherited ataxia affecting 1:50,000 Caucasians is characterised by neurodegeneration, cardiomyopathy, diabetes mellitus and skeletal deformities (Pandolfo M., "Friedreich Ataxia", Arch Neurol., 2008, 10, 1296-1303).

### Animal Model

To investigate FRDA molecular disease mechanisms and therapy, a human FXN YAC transgenic mouse model was established: YG8sR, which was bred from YG8R (described by Anjomani Virmouni, S., "Cellular, molecular and functional characterisation of YAC transgenic mouse models of Friedreich ataxia", PLoS One, 2014, 9, 1-13) to contain 120 to 220 GAA repeat sequences.

Use of a TaqMan qPCR assay to determine the FXN transgene copy number in the YG8R mouse line demonstrated that the YG8R line had two copies of the FXN transgene. The YG8sR mouse line showed less than one copy of the FXN transgene, suggesting potential deletion of one copy of the FXN transgene. Single integration sites of all transgenes were confirmed by fluorescence in situ hybridisation (FISH) analysis of metaphase and interphase chromosomes (see Anjomani Virmouni, S., 2013, Brunel University School of Health Sciences and Social Care PhD Thesis "Genotype and phenotype characterisation of Friedreich ataxia mouse models and cells", http://bura.brunel.ac.uk/handle/2438/7831)

### Method

Male and female mice were used and were 4-5 months old at the time of testing. The beam walk test was carried out using a 90cm long, 22mm diameter, wooden beam. The beam was placed horizontally 50cm above the bench surface with one end mounted on a narrow support with a 60W lamp, while a darkened escape box was located at the other end of the beam. Coordination ability was assessed by measuring the time taken for the mice to cross the beam and enter the escape box. Mice received two initial training sessions and were then assessed four times on their ability to traverse the beam (Test 1). The latency for the mice to traverse the beam was recorded. Mice were then orally administered with vehicle (an aqueous solution of 1% polyoxyethylenesorbitan monooleate (commercially sold under the trade mark "Tween 80")/0.5% methyl cellulose at a dose volume of 10mL/kg) or with a DAAO inhibitor being a compound of formula (I) above (at a dosage of 0.3, 1.0 or 3.0 mg/kg suspended in the above vehicle and at a dose volume of 10 mL/kg) and returned to their home cages. Five hours later the mice were assessed again four times on their ability to traverse the beam and the latency to traverse the beam was recorded (Test 2).

Four groups, each containing ten mice, were tested:
Group A was administered with vehicle alone and represented the control group.
Group B was administered the DAAO inhibitor at a dose of 0.3 mg/kg.
Group C was administered the DAAO inhibitor at a dose of 1.0 mg/kg.
Group D was administered the DAAO inhibitor at a dose of 3.0 mg/kg.

The results obtained are shown in Table 1 following:

**Table 1**

| Test No. | Average latency to cross beam (seconds) | | | |
|---|---|---|---|---|
| | Group A | Group B | Group C | Group D |
| 1 | 5.7 | 6.1 | 5.5 | 6.9 |
| 2 | 5.4 | 5.1 | 4.6 | 4.5 |

A statistical analysis of the results showed a significant improvement in performance of the mice treated with the DAAO inhibitor of formula (I) compared to mice treated with vehicle alone, at 3 mg/kg (p<0.05).

### Example 2

### Delayed Eyeblink Conditioning Test

Eyeblink conditioning (EBC) is a form of classical conditioning that has been used extensively to study neural structures and mechanisms that underlie learning and memory. The procedure consists of pairing an auditory or visual stimulus (the conditioned stimulus (CS)) with an eyeblink-eliciting unconditioned stimulus (US) (e.g. a mild puff of air to the cornea or a mild shock), for example, as described by Weeks, A. et al., "Eye-blink classical conditioning is associated with changes in synaptic ultrastructure in the interpositus nuclei of the rabbit cerebellum", Learning & Memory, 2007, 14, 385-389.

Naive animals initially produce a reflexive, unconditioned response (UR) (e.g. blink or extension of nictitating membrane) that follows US onset. After many CS-US pairings, an association is formed such that a learned blink, or conditioned response (CR), occurs and precedes US onset.

There are two experimental EBC procedures; delay and trace. In delay EBC (dEBC), the CS onset precedes the US onset and the two stimuli overlap and co-terminate. In the trace EBC (tEBC), the CS precedes the US and there is a stimulus free period (trace interval) between CS offset and US onset. While both of these procedures require the cerebellum, the trace procedure also requires the hippocampus (see, for example, Takehara, K., "Time-dependent reorganization of the brain components underlying memory retention in trace eyeblink conditioning", J. Neurosci., 2003, 23, 9896-9905 and Squire, L. R., "The medial temporal lobe", Annu. Rev. Neurosci., 2004, 27, 279-306).

The first evidence for the role of the cerebellum in EBC came from McCormick, D.A. et al, "Cerebellum: essential involvement in the classically conditioned eyelid response", Science, 1984, 223, 296-299. They found that a unilateral cerebellar lesion which included both cortex and deep nuclei permanently abolished CRs.

Impaired learning of conditioned eyeblink responses is a stable finding across multiple sessions in patients with degenerative cerebellar disorders, including spinocerebellar ataxia type 6 (SCA6), type 3 (SCA3), and Friedreich's ataxia (FRDA) (see Timmann, D., "Eyeblink conditioning in patients with hereditary ataxia: a one-year follow-up study", Exp Brain Res, 2005, 162(3), 332-45).

### dEBC Method

Three-month old, male C57B16 mice were implanted with recording electrodes in the *orbicularis oculi* muscle and with stimulating electrodes on the supraorbital nerve. For classical conditioning, animals were presented with a 350-ms tone as a conditioned stimulus (CS) at the end of which received an electrical pulse in the supraorbital nerve as unconditioned stimulus (US). Classical conditioning was achieved using a delay paradigm. For this, a tone (350 ms, 2 kHz, 85-90 dB) was presented as CS. The US consisted of a paired pulse with 1 millisecond of inter-pulse interval. Each pulse lasted for 0.1 millisecond. The US was presented at the end of the CS. A total of two habituation, ten conditioning, and five extinction sessions were carried out for each animal. Further details of this method can be found in the article by Gruart A., "Involvement of the CA3-CA1 synapse in the acquisition of associative learning in behaving mice", J. Neurosci., 2006, 26, 1077-1087.

The effects on associative learning of a DAAO inhibitor compound of the invention were compared against different control and scopolamine-administered groups. Scopolamine is a non-selective muscarinic receptor antagonist shown to consistently produce impairment of learning in rodents (see Klinkenberg, A., "The validity of scopolamine as a pharmacological model for cognitive impairment: A review of animal behavioral studies" Neuroscience and Biobehavioral Reviews, 2010, 34, 1307-1350.

Nine groups, each containing 15 mice except for the untreated control group which contained 14 mice, were tested:
**Group A** was orally administered with Vehicle 1 (an aqueous solution of 0.5% methyl cellulose);
**Group B** was orally administered with a solution of a DAAO inhibitor of formula (I) in Vehicle 1 at a dose of 0.1 mg/kg (Solution 1);
**Group C** was orally administered with a solution of a DAAO inhibitor of formula (I) (the same one as for Group B) in Vehicle 1 at a dose of 1.0 mg/kg (Solution 2);
**Group D** was administered subcutaneously with Vehicle 2 (distilled water);
**Group E** was administered subcutaneously with a solution of scopolamine in Vehicle 2 at a dose of 0.3 mg/kg (Solution 3);
**Group F** was administered orally with Solution 1 and subcutaneously with Solution 3;
**Group G** was administered orally with Solution 2 and subcutaneously with Solution 3;
**Group H** was administered orally with Vehicle1 and subcutaneously with Vehicle 2;
**Group I** was the untreated control group.

The results obtained are shown in Table 2 following.

**Table 2**

| Group | Mean Percentage of Conditioned Responses (%) | | | | | | | | | | | | | | | | | | |
|---|---|---|---|---|---|---|---|---|---|---|---|---|---|---|---|---|---|---|---|
| | Habituation | | | Conditioning | | | | | | | | | | | Extinction | | | | |
| A | 24.14 | 21.94 | 37.16 | | 47.00 | 59.78 | 58.89 | 59.67 | 65.67 | 67.89 | 70.92 | 74.33 | 73.79 | 60.09 | | 56.43 | 52.76 | 50.33 | 45.33 |
| B | 27.56 | 24.89 | 42.60 | | 51.78 | 57.89 | 63.11 | 65.56 | 66.00 | 68.89 | 68.89 | 76.78 | 77.74 | 65.50 | | 54.22 | 51.60 | 50.00 | 48.67 |
| C | 19.66 | 26.22 | 48.44 | | 59.56 | 65.22 | 71.00 | 74.67 | 73.44 | 76.00 | 75.78 | 80.33 | 79.20 | 63.11 | | 58.33 | 52.22 | 46.78 | 46.89 |
| D | 23.33 | 24.22 | 39.33 | | 49.11 | 59.67 | 61.00 | 61.44 | 66.56 | 72.14 | 75.44 | 75.78 | 76.22 | 64.78 | | 54.33 | 52.56 | 49.44 | 49.33 |
| E | 26.44 | 27.44 | 37.67 | | 40.33 | 43.78 | 43.33 | 45.67 | 45.98 | 48.78 | 51.56 | 51.33 | 52.56 | 51.22 | | 45.78 | 43.78 | 42.00 | 43.22 |
| F | 24.66 | 32.22 | 42.22 | | 45.11 | 51.22 | 52.89 | 54.89 | 55.00 | 57.67 | 58.67 | 57.89 | 58.22 | 49.56 | | 45.11 | 45.33 | 44.00 | 40.11 |
| G | 26.00 | 25.67 | 47.78 | | 51.33 | 58.89 | 61.11 | 60.11 | 67.74 | 73.11 | 72.89 | 71.22 | 76.11 | 61.33 | | 54.56 | 49.78 | 44.33 | 43.18 |
| H | 25.22 | 29.44 | 43.33 | | 51.11 | 60.33 | 61.67 | 64.00 | 70.44 | 74.11 | 76.33 | 78.67 | 81.44 | 62.33 | | 59.11 | 52.44 | 50.22 | 46.56 |
| I | 26.67 | 25.60 | 44.29 | | 50.12 | 61.94 | 65.00 | 64.05 | 70.55 | 77.46 | 77.14 | 81.43 | 82.50 | 67.02 | | 57.98 | 53.69 | 49.64 | 47.98 |

The data in Table 2 shows that administration of the DAAO inhibitor compound of formula (I) at a dose of 1 mg/kg (Group C) improved the rate of acquisition of the conditioned eyelid response relative to untreated and vehicle-treated control groups (Groups A, D H and I). Furthermore, administration of the DAAO inhibitor of formula (I) at a dose of 1 mg/kg significantly reversed the effects of scopolamine (0.3 mg/kg) administration on the generation of conditioned eyelid responses (compare Groups E and G with, for example, Group I).

## Claims

1. A compound of formula (I), or a pharmaceutically acceptable salt thereof, 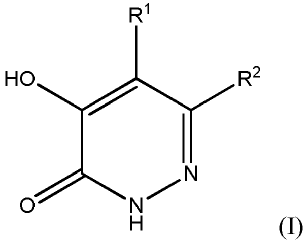 wherein
R¹ represents a hydrogen or fluorine atom or a trifluoromethyl group;
R² represents a group -X-Y-R³;
X and Y each independently represent a bond, an oxygen atom or a group - C(O), -S(O)ₙ, -C(O)NR⁴, -S(O)₂NR⁴, -NR⁴, 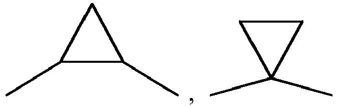 or -CR⁴R⁵-, provided that X and Y cannot both simultaneously represent a bond and provided that if X and Y are both other than a bond, then at least one of X and Y represents -CR⁴R⁵-;
n is 0, 1 or 2;
each R⁴ independently represents a hydrogen atom or a C₁-C₆ alkyl or C₁-C₆ haloalkyl group;
each R⁵ independently represents a hydrogen atom, a C₁-C₆ alkyl or C₁-C₆ haloalkyl group or =CH-;
R³ represents a 3- to 10-membered saturated or unsaturated carbocyclic or heterocyclic ring system, the ring system itself being optionally substituted by at least one substituent selected from halogen, hydroxyl, cyano, oxo, C₁-C₆ alkyl, C₂-C₆ alkenyl, C₁-C₆ haloalkyl, C₁-C₆ hydroxyalkyl, C₁-C₆ alkoxy, C₁-C₆ haloalkoxy, C₁-C₆ alkylthio, C₁-C₆ alkylsulphinyl, C₁-C₆ alkylsulphonyl, C₁-C₆ alkylcarbonyl, C₁-C₆ alkylcarbonyloxy, C₁-C₆ alkoxycarbonyl, amino, -CON(R⁶)₂, C₁-C₆ alkylamino, di-(C₁-C₆ alkyl)amino, C₃-C₆ cycloalkyl, C₃-C₆ cycloalkyloxy, C₃-C₆ cycloalkylmethyl, -[O]ₚ-(CH₂)_{q}-O-R⁷ and a 4- to 6-membered saturated or unsaturated heterocyclic ring (optionally substituted with at least one substituent selected from C₁-C₄ alkyl and C₁-C₄ alkoxy);
each R⁶ independently represents a hydrogen atom or a C₁-C₆ alkyl group;
p is 0 or 1;
q is 1, 2, 3 or 4; and
R⁷ represents a C₁-C₆ alkyl group;
for use in the prevention or treatment of an ataxic disorder.

2. A compound for use according to claim 1 which is used for the prevention or treatment of a spinocerebellar ataxic disorder or Friedreich's ataxia.

3. A compound for use according to claim 1 or claim 2 selected from
4-Hydroxy-6-(2-phenylethyl)pyridazin-3(2H)-one;
6-[2-(4-Fluorophenyl)ethyl]-4-hydroxypyridazin-3(2H)-one;
4-Hydroxy-6-{2-[5-(trifluoromethyl)pyridin-2-yl]ethyl}pyridazin-3(2H)-one;
6-[(4-Chlorobenzyl)sulfanyl]-4-hydroxypyridazin-3(2H)-one;
4-Hydroxy-6-{2-[6-(trifluoromethyl)pyridin-3-yl]ethyl}pyridazin-3(2H)-one;
6-[2-(3-Fluorophenyl)ethyl]-4-hydroxypyridazin-3(2H)-one;
6-[2-(2-Fluorophenyl)ethyl]-4-hydroxypyridazin-3(2H)-one;
6-[2-(3,5-Difluorophenyl)ethyl]-4-hydroxypyridazin-3(2H)-one;
6-[2-(3,4-Difluorophenyl)ethyl]-4-hydroxypyridazin-3(2H)-one;
4-Hydroxy-6-{2-[3-(trifluoromethoxy)phenyl]ethyl}pyridazin-3(2H)-one;
4-Hydroxy-6-{2-[3-(trifluoromethyl)phenyl]ethyl}pyridazin-3(2H)-one;
4-Hydroxy-6-{2-[5-(trifluoromethyl)pyridin-3-yl]ethyl}pyridazin-3(2H)-one;
6-(2-Cyclohexylethyl)-4-hydroxypyridazin-3(2H)-one;
6-(2-Cyclopropylethyl)-4-hydroxypyridazin-3(2H)-one;
6-(2-Cyclopentylethyl)-4-hydroxypyridazin-3(2H)-one;
4-Hydroxy-6-[2-(4-methoxycyclohexyl)ethyl]pyridazin-3(2H)-one;
6-[2-(2,4-Difluorophenyl)ethyl]-4-hydroxypyridazin-3(2H)-one;
6-{2-[3-(Difluoromethyl)phenyl]ethyl}-4-hydroxypyridazin-3(2H)-one;
6-Benzyl-4-hydroxypyridazin-3(2H)-one;
6-[2-(3-Chlorophenyl)ethyl]-4-hydroxypyridazin-3(2H)-one;
4-Hydroxy-6-(1-phenylcyclopropyl)pyridazin-3(2H)-one;
4-[2-(5-Hydroxy-6-oxo-1,6-dihydropyridazin-3-yl)ethyl]benzonitrile;
6-[2-(3-Fluoro-4-methylphenyl)ethyl]-4-hydroxypyridazin-3(2H)-one;
6-[2-(4-Fluoro-3-methylphenyl)ethyl]-4-hydroxypyridazin-3(2H)-one;
6- [2-(3,4-Dimethoxyphenyl)ethyl] -4-hydroxypyridazin-3 (2H)-one;
6-[2-(4-Chlorophenyl)ethyl]-4-hydroxypyridazin-3(2H)-one;
6-[2-(2-Chlorophenyl)ethyl]-4-hydroxypyridazin-3(2H)-one;
4-Hydroxy-6-{2-[2-(trifluoromethyl)phenyl]ethyl}pyridazin-3(2H)-one;
6-(4-(Difluoromethoxy)phenethyl)-4-hydroxypyridazin-3(2H)-one;
6-(4-(Trifluoromethoxy)phenethyl)-4-hydroxypyridazin-3(2H)-one;
6-(3 -(Difluoromethoxy)phenethyl)-4-hydroxypyridazin-3 (2H)-one;
6-[1-(4-Fluorophenyl)cyclopropyl]-4-hydroxypyridazin-3(2H)-one;
6-[1-(4-Fluorophenyl)ethyl]-4-hydroxypyridazin-3(2H)-one;
4-Hydroxy-6-{1-[3-(trifluoromethyl)phenyl]ethyl}pyridazin-3(2H)-one;
4-Hydroxy-6-{2-[4-(trifluoromethyl)phenyl]ethyl}pyridazin-3(2H)-one;
6-((Cyclopropylmethyl)(methyl)amino)-4-hydroxypyridazin-3(2H)-one;
6-((Cyclohexylmethyl)(methyl)amino)-4-hydroxypyridazin-3(2H)-one;
6-(3-Chlorobenzyl)-4-hydroxypyridazin-3(2H)-one;
6-(4-Chlorobenzyl)-4-hydroxypyridazin-3(2H)-one;
6-(Cyclohexylmethyl)-4-hydroxypyridazin-3(2H)-one;
6-(4-Fluorobenzyl)-4-hydroxypyridazin-3(2H)-one;
6-(2-Chloro-6-fluorobenzyl)-4-hydroxypyridazin-3(2H)-one;
6-(2-Chlorobenzyl)-4-hydroxypyridazin-3(2H)-one;
6-(3-Fluorobenzyl)-4-hydroxypyridazin-3(2H)-one;
6-(2-Fluorobenzyl)-4-hydroxypyridazin-3(2H)-one;
6-(4-Methylbenzyl)-4-hydroxypyridazin-3(2H)-one;
6-(3-Methylbenzyl)-4-hydroxypyridazin-3(2H)-one;
4-Hydroxy-6-(3-(trifluoromethyl)benzyl)pyridazin-3(2H)-one;
4-Hydroxy-6-[2-(oxan-4-yl)ethyl]pyridazin-3(2H)-one;
6-{[(4-Fluorophenyl)methyl](methyl)amino}-4-hydroxy-pyridazin-3(2H)-one;
6-[2-(2,6-Difluorophenyl)ethyl]-4-hydroxy-pyridazin-3(2H)-one;
6-[2-(2-Chloro-6-fluorophenyl)ethyl]-4-hydroxy-pyridazin-3(2H)-one;
6-{[3,5-bis(Trifluoromethyl)phenyl]methyl}-4-hydroxypyridazin-3(2H)-one;
6-(1-Phenylethyl)-4-hydroxypyridazin-3(2H)-one;
6-(Cyclopropylmethyl)-4-hydroxy-2,3-dihydropyridazin-3-one;
4-Hydroxy-6-{1-[4-(trifluoromethyl)phenyl]cyclopropyl}-2,3-dihydropyridazin-3-one;
6-{2-[2-Chloro-4-(trifluoromethyl)phenyl]ethyl}-4-hydroxy-2,3-dihydropyridazin-3-one;
6-{2-[2-Fluoro-4-(trifluoromethyl)phenyl]ethyl}-4-hydroxy-2,3-dihydropyridazin-3-one;
6-{2-[3,5-bis(Trifluoromethyl)phenyl]ethyl}-4-hydroxy-2,3-dihydropyridazin-3-one;
6-{2-[2,4-bis(Trifluoromethyl)phenyl]ethyl}-4-hydroxy-2,3-dihydro-pyridazin-3-one;
6-{2-[3,4-bis(Trifluoromethyl)phenyl]ethyl}-4-hydroxy-2,3-dihydropyridazin-3-one;
4-Hydroxy-6-(3-methyl-4-(trifluoromethyl)phenethyl)pyridazin-3(2H)-one;
4-Hydroxy-6-{2-[2-methyl-4-(trifluoromethyl)phenyl]ethyl}-2,3-dihydropyridazin-3-one;
6-{2-[3,5-Difluoro-4-(trifluoromethyl)phenyl]ethyl}-4-hydroxy-2,3-dihydropyridazin-3-one;
6-{2-[3-Fluoro-4-(trifluoromethyl)phenyl]ethyl}-4-hydroxy-2,3-dihydropyridazin-3-one;
and pharmaceutically acceptable salts thereof.

4. A compound of formula (I) for use according to any one of claims 1 to 3 which is 6-[2-(4-Fluorophenyl)ethyl]-4-hydroxypyridazin-3(2H)-one.

5. A compound of formula (I) for use according to any one of claims 1 to 3 which is 4-Hydroxy-6-{2-[4-(trifluoromethyl)phenyl]ethyl}pyridazin-3(2H)-one.

6. A compound of formula (I) for use according to any one of claims 1 to 3 which is 6-(4-Chlorobenzyl)-4-hydroxypyridazin-3(2H)-one.

7. A compound of formula (I) for use according to any one of claims 1 to 3 which is 6-(2-Fluorobenzyl)-4-hydroxypyridazin-3(2H)-one.

8. A compound of formula (I) or a pharmaceutically acceptable salt thereof as defined in any one of claims 1 and 3 to 7 for use in reducing the risk of an ataxic disorder.

9. A pharmaceutical composition comprising a compound of formula (I) or a pharmaceutically acceptable salt thereof as defined in any one of claims 1 and 3 to 7 in association with a pharmaceutically acceptable adjuvant, diluent or carrier, for use in the prevention or treatment of an ataxic disorder.

10. A pharmaceutical composition for use according to claim 9 which further comprises D-serine, D-serine ethyl ester, D-cycloserine, amantadine, amantadine hydrochloride, buspirone, acetazolamide, topiramate, divalproex sodium, L-dopa, propranolol, primidone, clonazepam, levetiracetam, carbamazepine, gabapentin, baclofen, ondansetron, tizanidine or pramipexole.

11. A pharmaceutical composition comprising a compound of formula (I) or a pharmaceutically acceptable salt thereof as defined in any one of claims 1 and 3 to 7 in association with a pharmaceutically acceptable adjuvant, diluent or carrier, for use in reducing the risk of an ataxic disorder.

12. A pharmaceutical composition for use according to claim 11 wherein the pharmaceutical composition further comprises D-serine, D-serine ethyl ester, D-cycloserine, amantadine, amantadine hydrochloride, buspirone, acetazolamide, topiramate, divalproex sodium, L-dopa, propranolol, primidone, clonazepam, levetiracetam, carbamazepine, gabapentin, baclofen, ondansetron, tizanidine or pramipexole.

13. A compound for use according to any one of claims 1 to 7, or a pharmaceutical composition for use according to claim 9 or claim 10, wherein the treatment comprises a reduction in the symptoms associated with an ataxic disorder.

14. A compound or pharmaceutical composition for use according to claim 13, wherein the treatment provides an improvement of gait, balance, limb coordination and/or speech.

15. A compound for use according to any one of claims 1 to 7, or a pharmaceutical composition for use according to claim 9 or claim 10, wherein the treatment results in an increased period of time between episodes of the ataxic disorder.

## Patentansprüche

1. Verbindung der Formel (I) oder ein pharmazeutisch akzeptables Salz davon 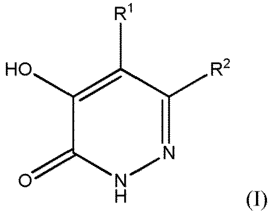 wobei
R¹ für ein Wasserstoff- oder Fluoratom oder eine Trifluormethylgruppe steht;
R² für eine Gruppe -X-Y-R³ steht;
X und Y jeweils unabhängig voneinanderfür eine Bindung, ein Sauerstoffatom oder eine Gruppe -C(O), -S(O)ₙ, -C(O)NR⁴, -S(O)₂NR⁴, -NR⁴, 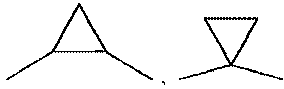 oder -CR⁴R⁵- stehen, vorausgesetzt, dass X und Y nicht beide gleichzeitig für eine Bindung stehen können, und vorausgesetzt, dass, wenn X und Y beide keine Bindung sind, mindestens entweder X oder Y für -CR⁴R⁵- steht;
n 0, 1 oder 2 ist;
jedes R⁴ unabhängig voneinander für ein Wasserstoffatom oder eine C₁-C₆-Alkyl- oder C₁-C₆-Halogenalkylgruppe steht;
jedes R⁵ unabhängig voneinander für ein Wasserstoffatom, eine C₁-C₆-Alkyl- oder C₁-C₆-Halogenalkylgruppe oder =CH- steht;
R³ ein 3- bis 10-gliedriges gesättigtes oder ungesättigtes carbocyclisches oder heterocyclisches Ringsystem darstellt, wobei das Ringsystem selbst optional substituiert ist mit mindestens einem Substituenten ausgewählt aus Halogen, Hydroxy, Cyano, Oxo, C₁-C₆-Alkyl, C2-C6-Alkenyl, C₁-C₆-Halogenalkyl, C₁-C₆-Hydroxyalkyl, C₁-C₆-Alkoxy, C₁-C₆-Halogenalkoxy, C₁-C₆-Alkylthio, C₁-C₆-Alkylsulfinyl, C₁-C₆-Alkylsulfonyl, C₁-C₆-Alkylcarbonyl, C₁-C₆-Alkylcarbonyloxy, C₁-C₆-Alkoxycarbonyl, Amino, -CON (R⁶)₂, C₁-C₆-Alkylamino, Di-(C₁-C₆-alkyl) amino, C₃-C₆-Cycloalkyl, C₃-C₆-Cycloalkyloxy, C₃-C₆-Cycloalkylmethyl, -[O]ₚ-(CH₂)_{q}-O-R⁷, und ein 4- bis 6-gliedriger gesättigter oder ungesättigter heterocyclischer Ring (optional substituiert mit mindestens einem Substituenten ausgewählt aus C₁-C₄-Alkyl und C₁-C₄-Alkoxy);
jedes R⁶ unabhängig voneinander für ein Wasserstoffatom oder eine C₁-C₆-Alkylgruppe steht;
p 0 oder 1 ist;
q 1, 2, 3 oder 4 ist; und
R⁷ für eine C₁-C₆-Alkylgruppe steht;
zur Verwendung bei der Vorbeugung oder Behandlung einer ataxischen Störung.

2. Verbindung zur Verwendung nach Anspruch 1, die zur Vorbeugung oder Behandlung einer spinozerebellären Ataxie oder Friedreich-Ataxie verwendet wird.

3. Verbindung zur Verwendung nach Anspruch 1 oder Anspruch 2, ausgewählt aus
4-Hydroxy-6-(2-phenylethyl)pyridazin-3(2H)-on;
6-[2-(4-Fluorphenyl)ethyl]-4-hydroxypyridazin-3(2H)-on;
4-Hydroxy-6-{2-[5-(trifluormethyl)pyridin-2-yl]ethyl}pyridazin-3(2H)-on;
6-[(4-Chlorbenzyl)sulfanyl] -4-hydroxypyridazin-3(2H)-on;
4-Hydroxy-6-{2-[6-(trifluormethyl)pyridin-3-yl]ethyl}pyridazin-3(2H)-on;
6-[2-(3-Fluorphenyl)ethyl]-4-hydroxypyridazin-3(2H)-on;
6-[2-(2-Fluorphenyl)ethyl]-4-hydroxypyridazin-3(2H)-on;
6-[2-(3,5-Difluorphenyl)ethyl]-4-hydroxypyridazin-3(2H)-on;
6-[2-(3,4-Difluorphenyl)ethyl]-4-hydroxypyridazin-3(2H)-on;
4-Hydroxy-6-{2-[3-(trifluormethoxy)phenyl]ethyl}pyridazin-3(2H)-on;
4-Hydroxy-6-{2-[3-(trifluormethyl)phenyl]ethyl}pyridazin-3(2H)-on;
4-Hydroxy-6-{2-[5-(trifluormethyl)pyridin-3-yl]ethyl}pyridazin-3(2H)-on;
6-(2-Cyclohexylethyl)-4-hydroxypyridazin-3(2H)-on;
6-(2-Cyclopropylethyl)-4-hydroxypyridazin-3(2H)-on;
6-(2-Cyclopentylethyl)-4-hydroxypyridazin-3(2H)-on;
4-Hydroxy-6-[2-(4-methoxycyclohexyl)ethyl]pyridazin-3(2H)-on;
6-[2-(2,4-Difluorphenyl)ethyl]-4-hydroxypyridazin-3(2H)-on;
6-{2-[3-(Difluormethyl)phenyl]ethyl}-4-hydroxypyridazin-3(2H)-on;
6-Benzyl-4-hydroxypyridazin-3(2H)-on;
6-[2-(3-Chlorphenyl)ethyl]-4-hydroxypyridazin-3(2H)-on;
4-Hydroxy-6-(1-phenylcyclopropyl)pyridazin-3(2H)-on;
4-[2-(5-Hydroxy-6-oxo-1,6-dihydropyridazin-3-yl)ethyl]benzonitril;
6-[2-(3-Fluor-4-methylphenyl)ethyl]-4-hydroxypyridazin-3(2H)-on;
6-[2-(4-Fluor-3-methylphenyl)ethyl]-4-hydroxypyridazin-3(2H)-on;
6-[2-(3,4-Dimethoxyphenyl)ethyl]-4-hydroxypyridazin-3(2H)-on;
6-[2-(4-Chlorphenyl)ethyl]-4-hydroxypyridazin-3(2H)-on;
6-[2-(2-Chlorphenyl)ethyl]-4-hydroxypyridazin-3(2H)-on;
4-Hydroxy-6-{2-[2-(trifluormethyl)phenyl]ethyl}pyridazin-3(2H)-on;
6-(4-(Difluoromethoxy)phenethyl)-4-hydroxypyridazin-3(2H)-on;
6-(4-(Trifluormethoxy)phenethyl)-4-hydroxypyridazin-3(2H)-on;
6-(3-(Difluoromethoxy)phenethyl)-4-hydroxypyridazin-3(2H)-on;
6-[1-(4-Fluorphenyl)cyclopropyl]-4-hydroxypyridazin-3(2H)-on;
6-[1-(4-Fluorphenyl)ethyl]-4-hydroxypyridazin-3(2H)-on;
4-Hydroxy-6-{1-[3-(trifluormethyl)phenyl]ethyl}pyridazin-3(2H)-on;
4-Hydroxy-6-{2-[4-(trifluormethyl)phenyl]ethyl}pyridazin-3(2H)-on;
6-((Cyclopropylmethyl)(methyl)amino)-4-hydroxypyridazin-3(2H)-on;
6-((Cyclohexylmethyl)(methyl)amino)-4-hydroxypyridazin-3(2H)-on;
6-(3-Chlorbenzyl)-4-hydroxypyridazin-3(2H)-on;
6-(4-Chlorbenzyl)-4-hydroxypyridazin-3(2H)-on;
6-(Cyclohexylmethyl)-4-hydroxypyridazin-3(2H)-on;
6-(4-Fluorbenzyl)-4-hydroxypyridazin-3(2H)-on;
6-(2-Chlor-6-fluorbenzyl)-4-hydroxypyridazin-3(2H)-on;
6-(2-Chlorbenzyl)-4-hydroxypyridazin-3(2H)-on;
6-(3-Fluorbenzyl)-4-hydroxypyridazin-3(2H)-on;
6-(2-Fluorbenzyl)-4-hydroxypyridazin-3(2H)-on;
6-(4-Methylbenzyl)-4-hydroxypyridazin-3(2H)-on;
6-(3-Methylbenzyl)-4-hydroxypyridazin-3(2H)-on;
4-Hydroxy-6-(3-(trifluormethyl)benzyl)pyridazin-3(2H)-on;
4-Hydroxy-6-[2-(oxan-4-yl)ethyl]pyridazin-3(2H)-on;
6-{[(4-Fluorphenyl)methyl](methyl)amino}-4-hydroxypyridazin-3(2H)-on;
6-[2-(2,6-Difluorphenyl)ethyl]-4-hydroxypyridazin-3(2H)-on;
6-[2-(2-Chlor-6-fluorphenyl)ethyl]-4-hydroxypyridazin-3(2H)-on;
6-{[3,5-Bis(trifluormethyl)phenyl]methyl}-4-hydroxypyridazin-3(2H)-on;
6-(1-Phenylethyl)-4-hydroxypyridazin-3(2H)-on;
6-(Cyclopropylmethyl)-4-hydroxy-2,3-dihydropyridazin-3-on;
4-Hydroxy-6-{1-[4-(trifluormethyl)phenyl]cyclopropyl}-2,3-dihydropyridazin-3-on;
6-{2-[2-Chlor-4-(trifluormethyl)phenyl]ethyl}-4-hydroxy-2,3-dihydropyridazin-3-on;
6-{2-[2-Fluor-4-(trifluormethyl)phenyl]ethyl}-4-hydroxy-2,3-dihydropyridazin-3-on;
6-{2-[3,5-Bis(trifluormethyl)phenyl]ethyl}-4-hydroxy-2,3-dihydropyridazin-3-on;
6-{2-[2,4-Bis(trifluormethyl)phenyl]ethyl}-4-hydroxy-2,3-dihydropyridazin-3-on;
6-{2-[3,4-Bis(trifluormethyl)phenyl]ethyl}-4-hydroxy-2,3-dihydropyridazin-3-on;
4-Hydroxy-6-(3-methyl-4-(trifluormethyl)phenethyl)pyridazin-3(2H)-on;
4-Hydroxy-6-{2-[2-methyl-4-(trifluormethyl)phenyl]ethyl}-2,3-dihydropyridazin-3-on;
6-{2-[3,5-Difluor-4-(trifluormethyl)phenyl]ethyl}-4-hydroxy-2,3-dihydropyridazin-3-on;
6-{2-[3-Fluor-4-(trifluormethyl)phenyl]ethyl}-4-hydroxy-2,3-dihydropyridazin-3-on;
und pharmazeutisch akzeptablen Salzen davon.

4. Verbindung der Formel (I) zur Verwendung nach einem der Ansprüche 1 bis 3, wobei diese 6-[2-(4-Fluorphenyl)ethyl]-4-hydroxypyridazin-3(2H)-on darstellt.

5. Verbindung der Formel (I) zur Verwendung nach einem der Ansprüche 1 bis 3, wobei diese 4-Hydroxy-6-{2-[4-(trifluormethyl)phenyl]ethyl}pyridazin-3(2H)-on darstellt.

6. Verbindung der Formel (I) zur Verwendung nach einem der Ansprüche 1 bis 3, wobei diese 6-(4-Chlorbenzyl)-4-hydroxypyridazin-3(2H)-on darstellt.

7. Verbindung der Formel (I) zur Verwendung nach einem der Ansprüche 1 bis 3, wobei diese 6-(2-Fluorbenzyl)-4-hydroxypyridazin-3(2H)-on darstellt.

8. Verbindung der Formel (I) oder ein pharmazeutisch akzeptables Salz davon wie in einem der Ansprüche 1 sowie 3 bis 7 definiert, zur Verwendung bei der Verringerung des Risikos für eine ataxischen Störung.

9. Pharmazeutische Zusammensetzung, umfassend eine Verbindung der Formel (I) oder ein pharmazeutisch akzeptables Salz davon wie in einem der Ansprüche 1 sowie 3 bis 7 definiert, in Verbindung mit einem pharmazeutisch akzeptablen Adjuvans, Verdünnungsmittel oder Träger, zur Verwendung bei der Vorbeugung oder Behandlung einer ataxischen Störung.

10. Pharmazeutische Zusammensetzung zur Verwendung nach Anspruch 9, wobei diese weiterhin D-Serin, D-Serinethylester, D-Cycloserin, Amantadin, Amantadinhydrochlorid, Buspiron, Acetazolamid, Topiramat, Divalproex-Natrium, L-Dopa, Propranolol, Primidon, Clonazepam, Levetiracetam, Carbamazepin, Gabapentin, Baclofen, Ondansetron, Tizanidin oder Pramipexol umfasst.

11. Pharmazeutische Zusammensetzung, umfassend eine Verbindung der Formel (I) oder ein pharmazeutisch akzeptables Salz davon wie in einem der Ansprüche 1 sowie 3 bis 7 definiert, in Verbindung mit einem pharmazeutisch akzeptablen Adjuvans, Verdünnungsmittel oder Träger, zur Verwendung bei der Verringerung des Risikos einer ataxischen Störung.

12. Pharmazeutische Zusammensetzung zur Verwendung nach Anspruch 11, wobei die pharmazeutische Zusammensetzung weiterhin D-Serin, D-Serinethylester, D-Cycloserin, Amantadin, Amantadinhydrochlorid, Buspiron, Acetazolamid, Topiramat, Divalproex-Natrium, L-Dopa, Propranolol, Primidon, Clonazepam, Levetiracetam, Carbamazepin, Gabapentin, Baclofen, Ondansetron, Tizanidin oder Pramipexol umfasst.

13. Verbindung zur Verwendung nach einem der Ansprüche 1 bis 7 oder pharmazeutische Zusammensetzung zur Verwendung nach Anspruch 9 oder Anspruch 10, wobei die Behandlung eine Verringerung der mit einer ataxischen Störung verbundenen Symptome beinhaltet.

14. Verbindung oder pharmazeutische Zusammensetzung zur Verwendung nach Anspruch 13, wobei die Behandlung eine Verbesserung von Gangart, Gleichgewicht, Gliedmaßenkoordination und/oder Sprache erbringt.

15. Verbindung zur Verwendung nach einem der Ansprüche 1 bis 7 oder pharmazeutische Zusammensetzung zur Verwendung nach Anspruch 9 oder Anspruch 10, wobei die Behandlung zu einer verlängerten Zeitspanne zwischen Episoden der ataxischen Störung führt.

## Revendications

1. Composé de formule (I), 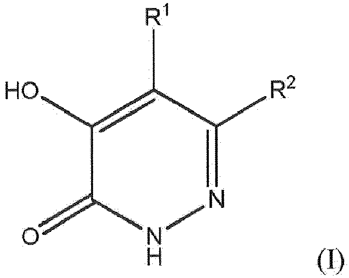 ou l'un de ses sels pharmaceutiquement acceptables :
formule dans laquelle
R¹ représente un atome d'hydrogène un atome de fluor ou un groupe trifluorométhyl,
R² représente un groupe X-Y-R³,
X et Y représentent chacun indépendamment l'un de l'autre, une liaison, un atôme d'oxygène ou un groupe
C(O), -S(O)ₙ, -C(O)NR⁴, -S(O)₂NR⁴, -NR⁴, 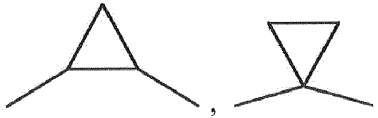
ou -CR⁴R⁵-, étant précisé que X et Y ne peuvent pas représenter simultanément une liaison, et que, si X et Y sont tous deux autres qu'une liaison, au moins l'un des X et Y représente -CR⁴R⁵-,
n est égal à 0, 1 ou 2,
chaque R⁴ représente indépendamment un atome d'hydrogène un groupe alkyl en C₁-C₆ ou un groupe halogéno-alkyl en C₁-C₆,
chaque R⁵ représente indépendamment un atome d'hydrogène, un groupe alkyl en C₁-C₆ ou un groupe halogéno-alkyl en C₁-C₆, ou =CH, R³ représente un système carbocyclique ou hétérocyclique saturé ou
insaturé ayant 3 à 10 côtés, ce système cyclique étant lui-même, le cas échéant substitué par au moins un substituant choisi parmi les substituants suivants : halogène hydroxy, cyano, oxo, alkyl en C₁-C₆, alkényl en C₂-C₆, halogéno-alkyl en C₁-C₆, hydroxyalkyl en C₁-C₆, alkoxy en C₁-C₆, halogéno-alkoxy en C₁-C₆, alkylthio en C₁-C₆, alkylsulfinyl en C₁-C₆,
alkylsulphonyl en C₁-C₆, alkylcarbonyl en C₁-C₆, alkylcarbonyloxy en C₁-C₆, alkoxycarbonyl en C₁-C₆, amino, -CON(R⁶)₂, alkylamino en C₁-C₆, di-C₁-C₆ alkylamino, cycloalkyl en C₃-C₆, cycloalkyloxy en C₃-C₆, cycloalkylméthyl en C₃-C₆, -[O]ₚ-(CH₂)q-OR⁷ et un héthérocycle saturé ou insaturé à 4 à 6 côtés, le cas échéant substitué par au moins un substituant choisi parmi les substituants suivants : alkyl en C₁-C₄ et alkoxy en C₁-C₄,
chaque R⁶ représente indépendamment un atome d'hydrogène ou un groupe alkyl en C₁-C₆,
p est égal à 0 ou 1,
q est égal à 3 ou 4, et
R⁷ représente un groupe alkyl en C₁-C₆,
destiné à être utilisé pour la prévention ou le traitement d'une ataxie.

2. Composé destine à être utilisé conformément à la revendication 1, utilisé pour la prévention et le traitement de l'ataxie spinocérébelleuse ou l'ataxie de Friedreich.

3. Composé destine à être utilisé conformément à la revendication 1 ou à la revendication 2,
choisi parmi les composés suivants :
4-Hydroxy-6-(2-phenylethyl)pyridazin-3(2H)-one;
6-[2-(4-Fluorophenyl)ethyl]-4-hydroxypyridazin-3(2H)-one;
4-Hydroxy-6-{2-[5-(trifluoromethyl)pyridin-2-yl]ethyl}pyridazin-3(2H)-one;
6-[(4-Chlorobenzyl)sulfanyl]-4-hydroxypyridazin-3(2H)-one;
4-Hydroxy-6-{2-[6-(trifluoromethyl)pyridin-3-yl]ethyl}pyridazin-3(2H)-one;
6-[2-(3-Fluorophenyl)ethyl]-4-hydroxypyridazin-3(2H)-one;
6-[2-(2-Fluorophenyl)ethyl]-4-hydroxypyridazin-3(2H)-one;
6-[2-(3,5-Difluorophenyl)ethyl]-4-hydroxypyridazin-3(2H)-one;
6-[2-(3,4-Difluorophenyl)ethyl]-4-hydroxypyridazin-3(2H)-one;
4-Hydroxy-6-{2-[3-(trifluoromethoxy)phenyl]ethyl}pyridazin-3(2H)-one;
4-Hydroxy-6-{2-[3-(trifluoromethyl)phenyl]ethyl}pyridazin-3(2H)-one;
4-Hydroxy-6-{2-[5-(trifluoromethyl)pyridin-3-yl]ethyl}pyridazin-3(2H)-one;
6-(2-Cyclohexylethyl)-4-hydroxypyridazin-3(2H)-one;
6-(2-Cyclopropylethyl)-4-hydroxypyridazin-3(2H)-one;
6-(2-Cyclopentylethyl)-4-hydroxypyridazin-3(2H)-one;
4-Hydroxy-6-[2-(4-methoxycyclohexyl)ethyl]pyridazin-3(2H)-one;
6-[2-(2,4-Difluorophenyl)ethyl]-4-hydroxypyridazin-3(2H)-one;
6-{2-[3-(Difluoromethyl)phenyl]ethyl}-4-hydroxypyridazin-3(2H)-one;
6-Benzyl-4-hydroxypyridazin-3(2H)-one;
6-[2-(3-Chlorophenyl)ethyl]-4-hydroxypyridazin-3(2H)-one;
4-Hydroxy-6-(1-phenylcyclopropyl)pyridazin-3(2H)-one;
4-[2-(5-Hydroxy-6-oxo-1,6-dihydropyridazin-3-yl)ethyl]benzonitrite;
6-[2-(3-Fluoro-4-methylphenyl)ethyl]-4-hydroxypyridazin-3(2H)-one;
6-[2-(4-Fluoro-3-methylphenyl)ethyl]-4-hydroxypyridazin-3(2H)-one;
6-[2-(3,4-Dimethoxyphenyl)ethyl]-4-hydroxypyridazin-3(2H)-one;
6-[2-(4-Chlorophenyl)ethyl]-4-hydroxypyridazin-3(2H)-one;
6-[2-(2-Chlorophenyl)ethyl]-4-hydroxypyridazin-3(2H)-one;
4-Hydroxy-6-{2-[2-(trifluoromethyl)phenyl]ethyl}pyridazin-3(2H)-one;
6-(4-(Difluoromethoxy)phenothyl)-4-hydroxypyridazin-3(2H)-one;
6-(4-(Trifluoromethoxy)phenethyl)-4-hydroxypyridazin-3(2H)-one;
6-(3-(Difluoromethoxy)phenethyl)-4-hydroxypyridazin-3(2H)-one;
6-[1-(4-Fluorophenyl)cyclopropyl]-4-hydroxypyridazin-3(2H)-one;
6-[1-(4-Fluorophenyl)ethyl]-4-hydroxypyridazin-3(2H)-one;
4-Hydroxy-6-{1-[3-(trifluoromethyl)phenyl]ethyl}pyriduzin-3(2H)-one;
4-Hydroxy-6-{2-[4-(trifluoromethyl)phenyl]ethyl}pyridazin-3(2H)-one;
6-((Cyclopropylmethyl)(methyl)amino)-4-hydroxypyridazin-3(2H)-one;
6-((Cyclohexylmethyl)(methyl)amino)-4-hydroxypyridazin-3(2H)-one;
6-(3-Chlorobenzyl)-4-hydroxypyridazin-3(2H)-one;
6-(4-Chlorobenzyl)-4-hydroxypyridazin-3(2H)-one;
6-(Cyclohexylmethyl)-4-hydroxypyridazin-3(2H)-one;
6-(4-Fluorobenzyl)-4-hydroxypyridazin-3(2H)-one;
6-(2-Chloro-6-fluorobenzyl)-4-hydroxypyridazin-3(2H)-onc;
6-(2-Chlorobenzyl)-4-hydroxypyridazin-3(2H)-one;
6-(3-Fluorobenzyl)-4-hydroxypyridazin-3(2H)-one;
6-(2-Fluorobenzyl)-4-hydroxypyridazin-3(2H)-one;
6-(4-Methylbenzyl)-4-hydroxypyridazin-3(2H)-one;
6-(3-Methylbenzyl)-4-hydroxypyridazin-3(2H)-one;
4-Hydroxy-6-(3-(trifluoromethyl)benzyl)pyridazin-3(2H)-one;
4-Hydroxy-6-[2-(oxan-4-yl)ethyl]pyridazin-3(2H)-one;
6-{[(4-Fluorophenyl)methyl](methyl)amino}-4-hydroxy-pyridazin-3(2H)-one;
6-[2-(2,6-Difluorophenyl)ethyl]-4-hydroxy-pyridazin-3(2H)-one;
6-[2-(2-Chloro-6-fluorophenyl)ethyl]-4-hydroxy-pyridazin-3(2H)-one;
6-{[3,5-bis(Trifluoromethyl)phenyl]methyl}-4-hydroxypyridazin-3(2H)-one;
6-(1-Phenylethyl)-4-hydroxypyridazin-3(2H)-one;
6-(Cyclopropylmethyl)-4-hydroxy-2,3-dihydropyridazin-3-one;
4-Hydroxy-6-{1-[4-(trifluoromethyl)phenyl]cyclopropyl}-2,3-dihydropyridazin-3-one;
6-{2-[2-Chloro-4-(trifluoromethyl)phenyl]ethyl}-4-hydroxy-2,3-dihydropyridazin-3-one;
6-{2-[2-Fluoro-4-(trifluoromethyl)phenyl]ethyl}-4-hydroxy-2,3-dihydropyridazin-3-one;
6-{2-[3,5-bis(Trifluoromethyl)phenyl]ethyl}-4-hydroxy-2,3-dihydropyridazin-3-one;
6-{2-[2,4-bis(Trifluoromethyl)phenyl]ethyl}-4-hydroxy-2,3-dihydro-pyridazin-3-one;
6-{2-[3,4-bis(Trifluoromethyl)phenyl]ethyl}-4-hydroxy-2,3-dihydropyridazin-3-one;
4-Hydroxy-6-(3-methyl-4-(trifluoromethyl)phenethyl)pyridazin-3(2H)-one;
4-Hydroxy-6-{2-[2-methyl-4-(trifluoromethyl)phenyl]ethyl}-2,3-dihydropyridazin-3-one;
6-{2-[3,5-Difluoro-4-(trifluoromethyl)phenyl]ethyl}-4-hydroxy-2,3-dihydropyridazin-3-one;
6-{2-[3-Fluoro-4-(trifluoromethyl)phenyl]ethyl}-4-hydroxy-2,3-dihydropyridazin-3-one;
et leurs sels pharmaceutiquement acceptables.

4. Composé de formule (I) destiné à être utilisé conformément à l'une quelconque des revendications 1 à 3, constitué par la 6-[2-(4-Fluorophenyl)ethyl]-4-hydroxypyridazin-3(2H)-one.

5. Composé de formule (I) destiné à être utilisé conformément à l'une quelconque des revendications 1 à 3, constitué par la 4-Hydroxy-6-{2-[4-trifluoromethyl)phényl]éthyl}pyridazine-3(2H)-one.

6. Composé de formule (I), destiné à être utilisé conformément à l'une quelconque des revendications 1 à 3, constitué par la 6-(4-Chlorobenzyl)-4-hydroxypyridazin-3(2H)-one.

7. Composé de formule (I), destiné être utilisé conformément à l'une quelconque des revendications 1 à 3, constitué par la 6-(2-Fluorobenzyl)-4-hydroxypyridazin-3(2H)-one.

8. Composé de formule (I) ou l'un de ses sels pharmaceutiquement acceptables conforme à l'une quelconque des revendications 1 et 3 à 7, destiné à être utilisé pour réduire le risque d'ataxie.

9. Composition pharmaceutique renfermant un composé de formule (I) ou l'un de ses sels pharmaceutiquement acceptable conforme à l'une quelconque des revendications 1 et 3 à 7, en association avec un adjuvant, un diluant ou un support pharmaceutiquement acceptable destinée à être utilisée pour la prévention ou le traitement d'une ataxie.

10. Composition pharmaceutique destinée à être utilisée conformément à la revendication 9, comprenant en outre l'un des composés suivants : D-serine, D-serine ethyl ester, D-cycloserine, amantadine, hydrochlorure d'amantadine, buspirone, acetazolamide, topiramate, divalproex sodium, L-dopa, propanolol, primidone, clonazepam, levetiracetam, carbamazepine, gabapentin, baclofen, ondansetron, tizanidine ou pramipexole.

11. Composition pharmaceutique renfermant un composé de formule (I) ou l'un de ses sels pharmaceutiquement acceptables conforme à l'une quelconque des revendications 1 et 3 à 7, en association avec un adjuvant, un diluant ou un support pharmaceutiquement acceptable destinée à être utilisée pour réduire le risque d'ataxie.

12. Composition pharmaceutique destinée à être utilisée conformément à la revendication 1, comprenant en outre l'un des composés suivants : D-serine, D-serine ethyl ester, D-cycloserine, amantadine, hydrochlorure d'amantadine, buspirone, acetazolamide, topiramate, divalproex sodium, L-dopa, propranolol, primidone, clonazepam, leveriracetam, carbamazepine, gabapentin, baclofen, ondansetron, tizanidine ou pramipexole.

13. Composé destiné à être utilisé conformément à l'une des revendications 1 à 7, ou composition pharmaceutique destinée à être utilisée conformément à la revendication 9 ou à la revendication 10, le traitement comprenant une réduction des symptômes associés à une ataxie.

14. Composé ou composition pharmaceutiquement acceptable destiné à être utilisé conformément à la revendication 13, dans lequel le traitement permet une amélioration de la démarche, de l'équilibre, de la coordination des membres et/ou de la parole.

15. Composé destiné à être utilisé conformément à l'une quelconque des revendications 1 à 7, ou composition pharmaceutique destinée à être utilisée conformément à la revendication 9 ou à la revendication 10, dans lequel le traitement a pour résultat une augmentation de la période de temps s'écoulant entre des épisodes d'ataxie.
